(19) Europäisches Patentamt
European Patent Office
Office européen des brevets

(11) Veröffentlichungsnummer: **0 475 156 A1**

(12) **EUROPÄISCHE PATENTANMELDUNG**

(21) Anmeldenummer: **91114138.0**

(22) Anmeldetag: **23.08.91**

(51) Int. Cl.[5]: **C07D 413/04**, A61K 31/415, A61K 31/445

(30) Priorität: **10.09.90 DE 4028679**

(43) Veröffentlichungstag der Anmeldung: **18.03.92 Patentblatt 92/12**

(84) Benannte Vertragsstaaten: **AT BE CH DE DK ES FR GB GR IT LI NL SE**

(71) Anmelder: **CASSELLA Aktiengesellschaft**
**Hanauer Landstrasse 526**
**W-6000 Frankfurt am Main 60(DE)**

(72) Erfinder: **Schönafinger, Karl, Dr.**
**Holunderweg 8**
**W-8755 Alzenau(DE)**
Erfinder: **Beyerle, Rudi, Dr.**
**An der Pfaffenmauer 44**
**W-6000 Frankfurt am Main 60(DE)**
Erfinder: **Bohn, Helmut, Dr.**
**Kranzbergring 11**
**W-6369 Schöneck(DE)**
Erfinder: **Just, Melitta, Dr.**
**Theodor-Heuss-Strasse 80**
**W-6070 Langen 2(DE)**

(74) Vertreter: **Urbach, Hans-Georg, Dr. et al**
**Hanauer Landstrasse 526**
**W-6000 Frankfurt am Main 61(DE)**

(54) **Substituierte 3-Aminosydnonimine, Verfahren zu ihrer Herstellung und ihre Verwendung.**

(57) Die Erfindung betrifft pharmakologisch wirksame substituierte 3-Aminosydnonimine der allgemeinen Formel I

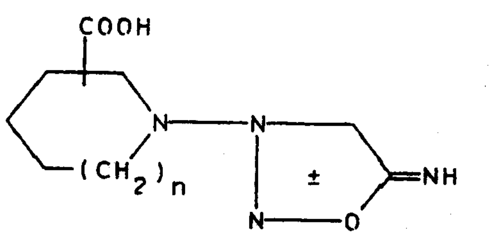

(I)

worin n 0 oder 1 bedeutet, und ihre pharmakologisch annehmbaren Salze und Säureadditionssalze, sowie ein Verfahren zur Herstellung der erfindungsgemäßen Verbindungen und ihre Verwendung.

EP 0 475 156 A1

Die Erfindung betrifft pharmakologisch wirksame substituierte 3-Aminosydnonimine der allgemeinen Formel I

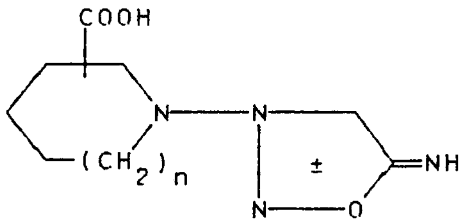

(I)

worin n 0 oder 1 bedeutet, und ihre pharmakologisch annehmbaren Salze und Säureddditionssalze.

Die Erfindung betrifft ferner ein Verfahren zur Herstellung der erfindungsgemäßen Verbindungen und ihre Verwendung.

In der allgemeinen Formel I befindet sich die Carboxylgruppe bevorzugt in der 2-, 3- oder 4-Position, besonders bevorzugt in der 2-Position, des heterocyclischen Substituenten. Dieser heterocyclische Substituent ist ein Pyrrolidin-Rest für n = 0 und ein Piperidin-Rest für n = 1.

In den Verbindungen der allgemeinen Formel I ist das die Carboxylgruppe tragende Kohlenstoffatom asymmetrisch. Somit können die erfindungsgemäßen Verbindungen als Racemat oder in Form ihrer Enantiomeren vorliegen.

Die vorliegende Erfindung betrifft sowohl das Racemat als auch das (S)- und das (R)-Enantiomere.

Bevorzugte Verbindungen der allgemeinen Formel I sind 3-(2-Carboxy-piperidino)-sydnonimin, (S)-3-(2-Carboxy-pyrrolidino)sydnonimin und 3-(4-Carboxy-piperidino)-sydnonimin. Besonders bevorzugt sind die Hydrochloride der genannten Verbindungen.

Eine Verbindung der allgemeinen Formel I kann dadurch hergestellt werden, daß eine Verbindung der allgemeinen Formel II

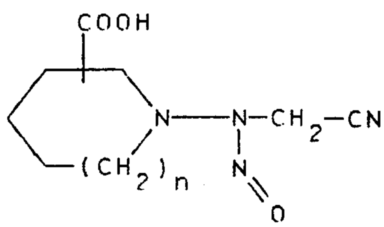

(II)

worin n 0 oder 1 bedeutet, cyclisiert und gegebenenfalls in ein pharmakologisch annehmbares Salz oder Säureadditionssalz überführt wird.

Die Cyclisierung der Verbindung II zu der Verbindung I wird in einem geeigneten organischen oder anorganischen Lösungs-, Dispergier- oder Verdünnungsmittel unter Zusatz eines Cyclisierungsmittels, normalerweise bei Temperaturen von -10 bis 40°C, insbesondere 0 bis 40°C, vorzugsweise bei 0 bis 20°C, durchgeführt.

Als Cyclisierungsmittel sind solche geeignet, die in wäßriger Lösung einen pH-Wert unter 3 einstellen, also z.B. Mineralsäuren, wie Schwefel-, Salpeter- oder Phosphorsäure, vorzugsweise Chlorwasserstoff, aber auch starke organische Säuren, wie Trifluoressigsäure. Die Cyclisierung wird normalerweise unter Eiskühlung durchgeführt.

Von dem Cyclisierungsmittel kommen z.B. bezogen auf 1 mol der Verbindung der Formel II 0,1 bis 10 mol, vorzugsweise 1 bis 5 mol, zur Anwendung. Das Cyclisierungsmittel wird normalerweise im Überschuß eingesetzt. Bei der Cyclisierung wird normalerweise das entsprechende Säureadditionssalz der Verbindung I erhalten.

Geeignete Lösungs-, Dispergier- oder Verdünnungsmittel sind z.B.: Alkohole, beispielsweise solche mit 1 bis 8 C-Atomen, insbesondere solche mit 1 bis 6 C-Atomen, vorzugsweise solche mit 1 bis 4 C-Atomen, wie z.B. Methanol, Ethanol, i- und n-Propanol, i-, sec- und tert-Butanol, n-, i-, sec-, tert-Pentanol, n-Hexanol, 2-Ethylbutanol, 2-Ethylhexanol, Isooctylalkohol, Cyclopentanol, Cyclohexanol, Methylcyclohexanol (Gemisch), Benzylalkohol; Ether, insbesondere solche mit 2 bis 8 C-Atomen im Molekül, wie z.B. Diethylether, Methyl-ethylether, Di-n-propyl-ether, Di-isopropyl-ether, Methyl-n-butylether, Methyl-tert-butylether, Ethyl-propyl-ether, Di-butyl-ether, Tetrahydrofuran, 1,4-Dioxan, 1,2-Dimethoxyethan, Bis-β-methoxyethylether; Oligoethylen-glykol-dimethylether, wie z.B. Tetraglyme oder Pentaglyme; Carbonsäurealkylester, insbesondere solche mit 2 bis 10 C-Atomen im Molekül, wie z.B. Ameisensäure-methyl-, -ethyl-, -butyl- oder -isobutyl-ester, Essigsäure-methyl-, -ethyl-, -propyl-, isopropyl-, -butyl-, -isobutyl- oder -sec-butyl-, -amyl-, -isoamyl-, -hexyl-, -cyclohexyl- oder -benzyl-ester, Propionsäure-methyl-, -ethyl- oder -butyl-ester; Ketone, insbesondere solche mit 3 bis 10 C-Atomen im Molekül, wie z.B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Methylcyclohexanon, Dimethylcyclohexanon, Benzophenon, Acetophenon; aliphatische Kohlenwasserstoffe, wie z.B. Hexan, Heptan, niedrig- und hochsiedende Petrolether, Spezialbenzine und Testbenzin; cycloaliphatische Kohlenwasserstoffe, wie z.B. Cyclopentan, Cyclohexan, Methylcyclohexan, Tetralin, Decalin; aromatische Kohlenwasserstoffe, wie z.B. Benzol, Toluol, o-, m- und p-Xylol, Ethylbenzol; halogenierte aliphatische oder aromatische Kohlenwasserstoffe, wie z.B. Methylenchlorid, Chloroform, Tetrachlorkohlenstoff, 1,2-Dichlorethan, Chlorbenzol, Dichlorbenzol; Hexamethylphosphorsäuretriamid; Sulfoxide, wie z.B. Dimethyl-sulfoxid; Tetramethylensulfon; Wasser. Auch Gemische verschiedener Lösungs- oder Dispergiermittel können verwendet werden, beispielsweise Wasser-Methanol oder vorzugs weise Essigsäureethylester-Methanol.

Die Verbindungen der allgemeinen Formel I können mit anorganischen oder organischen Säuren Säureadditionssalze bilden. Geeignete Säuren für die Bildung pharmakologisch annehmbarer Säureadditionssalze sind beispielsweise: Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipinsäure. Die Säureadditionssalze können wie üblich durch Vereinigung der Komponenten, zweckmäßigerweise in einem geeigneten Lösungs- oder Verdünnungsmittel, hergestellt werden.

Bei der Synthese der Verbindung der Formel I fallen normalerweise die Säureadditionssalze an.

Daneben können die Verbindungen der allgemeinen Formel I auch in Form ihrer inneren Salze vorliegen.

Die Ausgangsverbindungen der allgemeinen Formel II können in einfacher und an sich bekannter Weise nach folgendem Reaktionsschema hergestellt werden:

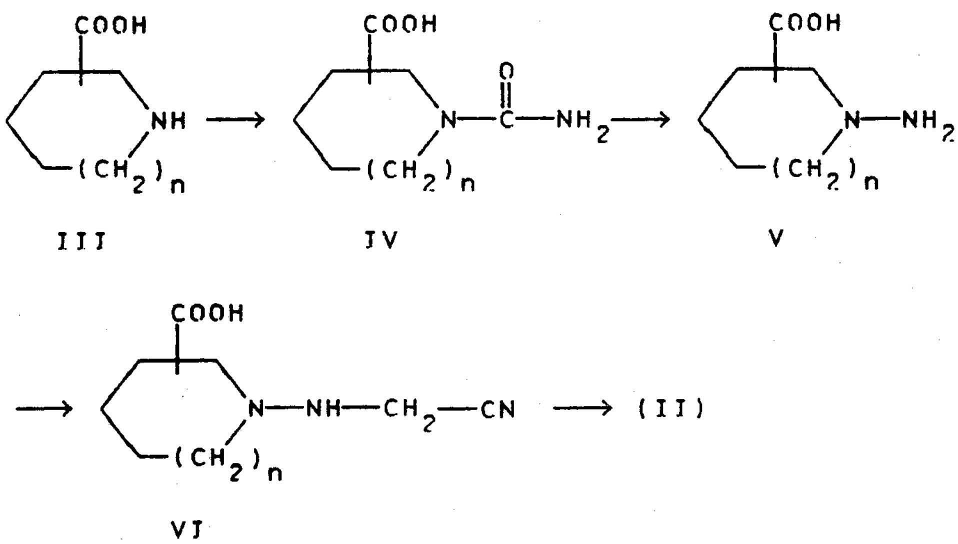

III IV V

VI (II)

Danach wird die heterocyclische Säure III beispielsweise mit Alkalicyanat in saurer wäßriger Lösung in das Harnstoffderivat IV und dieses durch Hofmann-Abbau in das Hydrazin V überführt. Alternativ kann die Verbindung der Formel V aber auch reduktiv aus der N-Nitrosoverbindung der Verbindung der Formel III hergestellt werden (für 1-Aminoprolin z.B. Biochemistry 6, 173 (1967)). Die Verbindung der Formel V wird schließlich zur Verbindung der Formel VI cyanmethyliert und diese zur Verbindung der Formel II nitrosiert.

Die Harnstoffsynthese durch Umsetzung eines Amins mit einem Alkalicyanat in saurer wäßriger Lösung ist bekannt und in der Literatur beschrieben (siehe z.B. Houben-Weyl, Methoden der Organischen Chemie, 4. Auflage Bd. E4, Seite 362). Ein bevorzugtes Alkalicyanat ist dabei Kaliumcyanat. Auch der Hofmann-Abbau, d.h. die Synthese eines Amins aus dem entsprechenden Amid durch Umsetzung mit Hypochlorit oder -bromit, ist literaturbekannt (siehe z.B. Houben-Weyl, Methoden der Organischen Chemie, 4. Auflage

Bd. XI/1 Seiten 854 ff.).

Die Cyanmethylierung zur Verbindung der Formel VI gelingt in ebenfalls bekannter Weise durch Umsetzung der Verbindung der Formel V mit Formaldehyd und Blausäure bzw. Natriumcyanid in einem geeigneten Lösungsmittel, z.B. Wasser. Schließlich wird auch die Nitrosierung in bekannter Weise, vorzugsweise in Wasser, z.B. bei Temperaturen von 0 bis 10°C durchgeführt. Die salpetrige Säure wird dabei normalerweise aus einem Alkalimetallnitrit, z.B. Natriumnitrit, und Salzsäure erzeugt. Es ist zweckmäßig, die wäßrige Lösung der Vorstufen mit Salzsäure auf einen pH-Wert von 1 bis 3 einzustellen und das Alkalimetallnitrit in Form einer wäßrigen Lösung zu der gerührten und abgekühlten Lösung der Verbindung zuzutropfen.

Die Verbindungen der allgemeinen Formel III sind bekannt und im Handel erhältlich und/oder nach bekannten Methoden herstellbar. Die Verbindungen der allgemeinen Formel III sind insbesondere Prolin, Pyrrolidin-3-carbonsäure, Pipecolinsäure, Nipecotinsäure und Isonipecotinsäure.

Werden die Verbindungen der allgemeinen Formel III in Form ihrer Racemate eingesetzt, so werden auch die erfindungsgemäßen Verbindungen der allgemeinen Formel I als Racemat erhalten. Entsprechend ist die Synthese der (S)- bzw. (R)-Formen der erfindungsgemäßen Verbindungen der allgemeinen Formel I durch Einsatz der entsprechenden enantiomerenreinen Verbindungen der allgemeinen Formel III möglich.

Die (S)- bzw. (R)-Formen der erfindungsgemäßen Verbindungen der allgemeinen Formel I sind daneben auch durch die bekannten Methoden zur Racemattrennung aus ihrem racemischen Gemisch zugänglich, beispielsweise durch Veresterung des Racemats mit einem optisch aktiven Alkohol, Trennung des entstandenen Diastereomerengemisches und Verseifung der Ester.

Die Verbindungen der allgemeinen Formel I und ihre pharmakologisch annehmbaren Salze und Säureadditionssalze besitzen wertvolle pharmakologische Eigenschaften. Sie sind in der Lage, unter physiologischen Bedingungen Stickstoffmonoxid freizusetzen und so die antiaggregatorische, antiadhäsive und muskelrelaxierende Wirkung des endothelium derived relaxing factor (EDRF) zu verstärken. Sie können also eingesetzt werden bei Krankheiten, wo die natürliche endotheliale NO-Freisetzung nicht ausreichend ist, wie z.B. Angina pectoris und Thrombosen.

Verglichen mit anderen Sydnoniminen zeigen die erfindungsgemäßen Verbindungen eine langsamere NO-Freisetzung, die zu einer gleichmäßigen und langen Wirkung führt.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Salzes oder Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Tragerstoffe für die Herstellung von Injektionslösungen eig nen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: $\beta$-Rezeptorenblocker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat,

Fenofibrat; Mittel für die Thromboseprophylaxe, wie z.B. Phenprocoumon.

Die Verbindungen der Formel I, ihre pharmakologisch annehmbaren Salze und Säureadditionsssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris und Thrombosen. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Die pharmakologische Wirkung der Verbindungen der Formel I wurde nach einer modifizierten Methode von Godfraind and Kaba (Arch.Int. Pharmacodyn. Ther. 196, (Suppl) 35 bis 49, 1972) und von Schüman et al (Naunyn-Schmiedeberg's Arch. Pharmacol. 289, 409 bis 418, 1975) ermittelt. Dabei werden Spiralstreifen der Arteria pulmonalis des Meerschweinchens nach Äquilibrierung in calciumfreier Tyrodelösung mit 40 mmol/l Kalium depolarisiert. Ein Zusatz von 0,5 mmol/l $CaCl_2$ löst dann eine Kontraktion aus.

Die relaxierende Wirkung der Prüfsubstanz wird durch kumulative Zugabe in 1/2 log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefäßstreifen) wird die Konzentration der Prüfsubstanz ermittelt, welche die Kontraktion um 50 % hemmt (= $IC_{50}$, mol/l). Die Wirkungsdauer der Prüfsubstanz ergibt sich aus der Zeit, die nach der Zugabe der Prüfsubstanz benötigt wird, bis der Ausgangswert wieder erreicht ist. In der folgenden Tabelle sind die so erhaltenen Werte angegeben.

| Verbindung aus | $IC_{50}$ | Wirkdauer in Minuten |
|---|---|---|
| Beispiel 1 | $6.10^{-6}$ | > 300 |
| Beispiel 2 | $2.4.10^{-5}$ | > 300 |
| Beispiel 3 | $2.10^{-5}$ | > 300 |
| Vergleichssubstanzen | | |
| SIN-1 | $1.10^{-6}$ | 90 |
| Molsidomin | $3.10^{-4}$ | 120 |

SIN-1 = N-Ethoxycarbonyl-3-morpholino-sydnonimin

Molsidomin = 3-Morpholino-sydnonimin-hydrochlorid

## Beispiele

### 1. 3-(2-Carboxy-piperidino)-sydnonimin-hydrochlorid

Die Mischung von 129 g (D,L)-Pipecolinsäure, 500 ml Wasser und 122 g Kaliumcyanat wird kurz bis zum Siedepunkt erhitzt und nach 5 Min. im Eisbad abgekühlt und mit 200 ml 50 %iger Schwefelsäure sauer gestellt. Der entstandene Harnstoff (1-Aminocarbonyl-piperidin-2-carbonsäure) wird abgesaugt und getrocknet.

Ausbeute: 149 g

Fp. 148°C (Zers.)

12,9 g dieser Verbindung und 12,6 g KOH werden in 40 ml Wasser gelöst, auf 5°C abgekühlt und mit 32,6 g einer 30 %igen Kaliumhypochlorit-Lösung versetzt und bei ansteigender Temperatur 15 Stunden gerührt. Durch Zugabe von 3 g Natriumsulfit wird das überschüssige Hypochlorit zerstört. Die Mischung wird filtriert, abgekühlt und mit 22,5 ml einer 10 M Salzsäure sauer gestellt. Nach Zufügen von 6,5 g

Kaliumcyanid wird mit Salzsäure auf pH = 7,6 gestellt und 5,9 g einer 38 %igen Formalinlösung zugesetzt. Das pH der Mischung wird mit Sodalösung auf 7 - 7,5 eingestellt und der Ansatz 15 Stunden bei Raumtemperatur gerührt. Die Mischung wird im Eisbad gekühlt, mit 10 M Salzsäure sauer gestellt (pH = 1), mit 6,9 g Natriumnitrit versetzt und 5 Stunden bei ansteigender Temperatur gerührt. Dann werden 100 ml Essigester zugesetzt und die wässrige Phase auf pH = 1 gestellt. Die Essigesterphase wird abgetrennt, über Natriumsulfat unter Stickstoff getrocknet und mit 25 ml einer 30 %igen isopropanolischen Salzsäure vermischt. Nach dem Stehenlassen über Nacht bei Raumtemperatur wird der ausgefallene Feststoff durch Filtrieren entfernt und das Filtrat eingeengt. Der ölige Rückstand wird in 50 ml Methanol gelöst und mit A-Kohle erwärmt, filtriert und mit 800 ml Essigester verdünnt. Aus dieser Lösung kristallisiert ein Feststoff nach einiger Zeit aus, der abgesaugt und getrocknet wird.
Ausbeute: 7,4 g
Fp. 122°C (Zers.)

2. 3-(4-Carboxy-piperidino)-sydnonimin-hydrochlorid

wurde in analoger Weise aus Isonipecotinsäure erhalten und schmilzt bei 172°C unter Zersetzung.

3. (S)-3-(2-Carboxy-pyrrolidino)-sydnonimin-hydrochlorid

wurde in analoger Weise aus L-Prolin erhalten.
Fp. 142 - 144°C (Zers.)
Drehwert: $\alpha_D^{20}$ = -100° (Wasser; C = 1,0)

**Patentansprüche**

1. Substituierte 3-Aminosydnonimine der allgemeinen Formel I

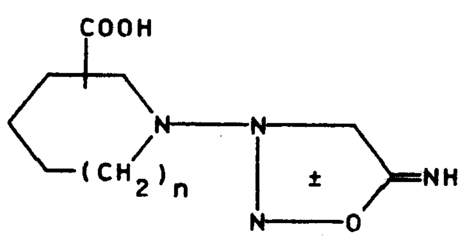

(I)

worin n 0 oder 1 bedeutet, und ihre pharmakologisch annehmbaren Salze und Säureadditionssalze.

2. Substituierte 3-Aminosydnonimine gemäß Anspruch 1, dadurch gekennzeichnet, daß sich die Carboxylgruppe in der 2-Position des heterocyclischen Substituenten befindet.

3. 3-(2-Carboxy-piperidino)-sydnonimin-Hydrochlorid.

4. (S)-3-(2-Carboxy-pyrrolidino)-sydnonimin-Hydrochlorid.

5. 3-(4-Carboxy-piperidino)-sydnonimin-Hydrochlorid.

6. Verfahren zur Herstellung eines substituierten 3-Amino-sydnonimins der allgemeinen Formel I gemäß einem oder mehreren der Ansprüche 1 bis 5, dadurch gekennzeichnet, daß eine Verbindung der allgemeinen Formel II

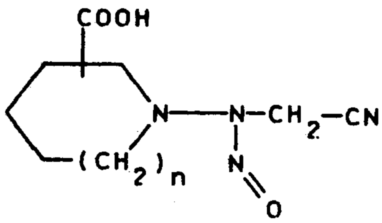

(II)

worin n 0 oder 1 bedeutet, cyclisiert und gegebenenfalls in ein pharmakologisch annehmbares Salz oder Säureadditionssalz überführt wird.

**7.** Verwendung substituierter 3-Aminosydnonimine der allgemeinen Formel I gemäß einem oder mehreren der Ansprüche 1 bis 5 oder pharmakologisch annehmbarer Salze oder Säureadditionssalze davon zur Herstellung von Arzneimitteln zur Vorbeugung und Bekämpfung von cardiovaskulären Erkrankungen.

**8.** Verwendung substituierter 3-Aminosydnonimine der allgemeinen Formel I gemäß einem oder mehreren der Ansprüche 1 bis 5 oder pharmakologisch annehmbarer Salze oder Säureadditionssalze davon zur Herstellung von Arzneimitteln zur Behandlung von Angina pectoris und zur Vorbeugung und Heilung von Thrombosen.

**9.** Pharmazeutisches Präparat, dadurch gekennzeichnet, daß es ein substituiertes 3-Aminosydnonimin der allgemeinen Formel I gemäß einem oder mehreren der Ansprüche 1 bis 5 oder ein pharmakologisch annehmbares Salz oder Säureadditionssalz davon als Wirkstoff zusammen mit pharmazeutisch annehmbaren Träger- und Zusatzstoffen und gegebenenfalls noch ein oder mehrere andere pharmakologische Wirkstoffe enthält.

**Patentansprüche für folgende Vertragsstaaten: ES, GR**

**1.** Verfahren zur Herstellung eines substituierten 3-Aminosydnonimins der allgemeinen Formel I

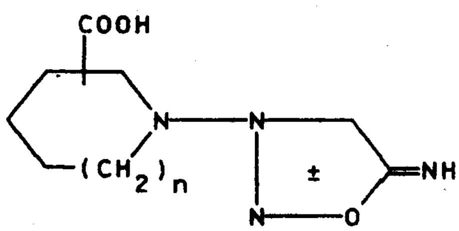

(I)

worin n 0 oder 1 bedeutet, oder eines pharmakologisch annehmbaren Salzes oder Säureadditionssalzes davon, dadurch gekennzeichnet, daß eine Verbindung der allgemeinen Formel II

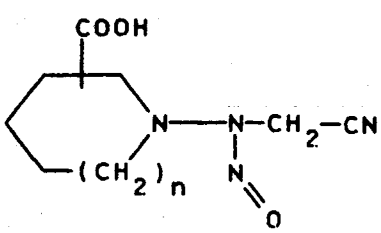

(II)

worin n 0 oder 1 bedeutet, cyclisiert und gegebenenfalls in ein pharmakologisch annehmbares Salz oder Säureadditionssalz überführt wird.

**2.** Verfahren gemäß Anspruch 1, dadurch gekennzeichnet, daß sich in der allgemeinen Formel I die Carboxylgruppe in der 2-Position des heterocyclischen Substituenten befindet.

3. Verfahren gemäß Anspruch 1 und/oder 2, dadurch gekennzeichnet, daß 3-(2-Carboxy-piperidino)-sydnonimin-Hydrochlorid hergestellt wird.

4. Verfahren gemäß Anspruch 1 und/oder 2, dadurch gekennzeichnet, daß (S)-3-(2-Carboxy-pyrrolidino)-sydnonimin-Hydrochlorid hergestellt wird.

5. Verfahren gemäß Anspruch 1, dadurch gekennzeichnet, daß 3-(4-Carboxy-piperidino)-sydnonimin-Hydrochlorid hergestellt wird).

6. Verwendung substituierter 3-Aminosydnonimine der allgemeinen Formel I gemäß einem oder mehreren der Ansprüche 1 bis 5 oder pharmakologisch annehmbarer Salze oder Säureadditionssalze davon zur Herstellung von Arzneimitteln zur Vorbeugung und Bekämpfung von cardiovaskulären Erkrankungen.

7. Verwendung substituierter 3-Aminosydnonimine der allgemeinen Formel I gemäß einem oder mehreren der Ansprüche 1 bis 5 oder pharmakologisch annehmbarer Salze oder Säureadditionssalze davon zur Herstellung von Arzneimitteln zur Behandlung von Angina pectoris und zur Vorbeugung und Heilung von Thrombosen.

8. Verfahren zur Herstellung eines pharmazeutischen Präparates enthaltend ein substituiertes 3-Aminosydnonimin der allgemeinen Formel I gemäß einem oder mehreren der Ansprüche 1 bis 5 oder ein pharmakologisch annehmbares Salz oder Säureadditionssalz davon, dadurch gekennzeichnet, daß man diese zusammen mit pharmazeutisch annehmbaren Träger- und Zusatzstoffen und gegebenenfalls noch einem oder mehreren anderen pharmakologischen Wirkstoffen in eine geeignete Darreichungsform bringt.

Europäisches Patentamt

# EUROPÄISCHER RECHERCHENBERICHT

Nummer der Anmeldung

EP 91114138.0

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
| A | FR - A - 1 551 013 (BOEHRINGER) * Ansprüche 1,2 * -- | 1,6 | C 07 D 413/04<br>A 61 K 31/415<br>A 61 K 31/445 |
| A | US - A - 3 312 690 (MASUDA) * Anspruch 1 * -- | 1 | |
| A | DE - A1 3 702 083 (CASSELLA) * Ansprüche 1,7 * -- | 1,9 | |
| A | CHEMICAL ABSTRACTS, Band 73, Nr. 1, 6. Juli 1970, Columbus, Ohio, USA KIKUCHI, KENZO et al. "Cardiovascular action of mesoionic compounds, 3-substituted sydnonimines." Seite 206, Spalte 1, Zusammenfassung-Nr. 2 425y & Jap. J. Pharmacol. 1970, 20(1), 23-43 -- | 1,7 | RECHERCHIERTE SACHGEBIETE (Int. Cl.⁵)<br>C 07 D 413/00 |
| A | DE - A1 - 3 819 914 (CASSELLA) * Ansprüche 1,9,10 * ---- | 1,7,9 | |

Der vorliegende Recherchenbericht wurde für alle Patentansprüche erstellt.

| Recherchenort | Abschlußdatum der Recherche | Prüfer |
|---|---|---|
| WIEN | 04-11-1991 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82